# EUROPEAN PATENT APPLICATION

(11) **EP 4 310 190 A1**
(43) Date of publication of application: **24.01.2024**
(21) Application number: 23778975.5
(22) Date of filing: 16.02.2023
(51) Int. Cl.: C12P 1/04, A61K 35/74, A61P 31/10, C12N 1/20

(54) **NOVEL ANTIFUNGAL AGENT**

(30) Priority: 30.03.2022 JP 2022055718
(71) Applicant: Murata Manufacturing Co., Ltd., Nagaokakyo-shi, Kyoto 617-8555 (JP)
(72) Inventor: TAKATA, Masachika, Nagaokakyo-shi, Kyoto 617-8555 (JP); SUNAHARA, Hirofumi, Nagaokakyo-shi, Kyoto 617-8555 (JP)
(74) Representative: Reeve, Nicholas Edward
(86) International application number: PCT/JP2023/005404
(87) International publication number: WO 2023/188945

(57) **Abstract**

Provided are an antifungal agent comprising an antifungal substance derived from a bacterium belonging to genus Acidipila, and a method for producing the same, and the like.

## Description

### TECHNICAL FIELD

The present invention relates to antifungal substances derived from bacteria of genus Acidipila.

### BACKGROUND ART

Of drugs approved worldwide from 1981 to 2019, more than half are derived from natural products, including derivative compounds having basic skeletons of natural products and synthetic compounds that mimic structures of natural products (Non-Patent Literature 1). A significant number of natural product-derived drugs are actually produced by microorganisms. Thus, in pharmaceutical development, it is very important to search for natural products derived from microorganisms.

Actinomycetes are a group of microorganisms that have received the most attention as a source of new drugs since the discovery of streptomycin. Actinomycetes are known as a group of bacteria that have many biosynthetic gene clusters related to secondary metabolites and produce secondary metabolites rich in chemical diversity. There are hundreds of actinomycete-derived antibiotics that have been put into practical use. Thus actinomycetes are the most important taxonomic group as a source of natural products.

Regarding drug research and development for diseases caused by microorganisms and fungi, however, there is a problem that the return on an investment in the research and development is disproportionate to costs for continuing the research and development. Thus, there are currently very few pharmaceutical companies that actively search for drugs derived from natural products. Especially, development of antifungal agents has a serious situation. Since 2006, new antifungal agents derived from natural products have not been reported. On the other hand, new infectious fungi have been discovered in patients. Thus development of new antifungal agents is demanded.

### CITATION LIST

### NON-PATENT LITERATURE

Non-patent Literature 1: J Nat Prod 2020 Mar 27; 83(3):770-803, "Natural Products as Sources of New Drugs over the Nearly Four Decades from 01/1981 to 09/2019"

### SUMMARY OF THE INVENTION

### PROBLEMS TO BE SOLVED BY THE INVENTION

An object of the present invention is to search for new groups of culturable microorganisms that produce secondary metabolites rich in diversity and to provide novel antifungal compounds.

### SOLUTION TO PROBLEMS

As described above, actinomycetes were mainly searched as sources of natural products for pharmaceuticals. The present inventors have newly focused on bacteria in the phylum Acidobacteria which have a high ability to produce secondary metabolites. Most bacteria in the phylum Acidobacteria are gram-negative bacteria existing in soil and are difficult to culture. Thus most of the bacteria in the phylum Acidobacteria have never been cultivated. As a result of intensive research, the present inventors surprisingly found that, in the phylum Acidobacteria, a group of bacteria belonging to the genus Acidipila produced antifungal substances. Furthermore, the present inventors isolated novel bacteria belonging to the genus Acidipila. Thus, the present invention was completed.

The present invention provides, for example, the following aspects.
[1] An antifungal agent, comprising an antifungal substance derived from a bacterium belonging to genus Acidipila and targeting a yeast or a filamentous fungus.
[2] The antifungal agent according to [1], wherein the bacterium belonging to genus Acidipila is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).
[3] The antifungal agent according to [1] or [2], wherein the filamentous fungus is selected from the group consisting of fungi of genus Aspergillus, fungi of genus Mucor, and fungi of genus Trichophyton.
[4] The antifungal agent according to [1] or [2], wherein the yeast is selected from the group consisting of fungi of genus Saccharomyces, fungi of genus Candida, fungi of genus Malassezia, and fungi of genus Rhodotorula.
[5] The antifungal agent according to any one of [1] to [4], wherein the antifungal substance derived from a bacterium belonging to genus Acidipila is a culture or a culture supernatant of the bacterium, or a concentrate or an extract of the culture or culture supernatant.
[6] A method for producing an antifungal agent targeting a yeast or a filamentous fungus, the method comprising culturing a bacterium belonging to genus Acidipila.
[7] The method according to [6], wherein the bacterium belonging to genus Acidipila is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).
[8] The method according to [6] or [7], wherein the filamentous fungus is selected from the group consisting of fungi of genus Aspergillus, fungi of genus Mucor, and fungi of genus Trichophyton.
[9] The method according to [6] or [7], wherein the yeast is selected from the group consisting of fungi of genus Saccharomyces, fungi of genus Candida, fungi of genus Malassezia, and fungi of genus Rhodotorula.
[10] A bacterium which is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).
[11] A method for inhibiting the growth of a fungus which is a yeast or a filamentous fungus, the method comprising bringing an antifungal substance derived from a bacterium belonging to genus Acidipila into contact with the yeast or filamentous fungus.
[12] The method according to [11], wherein the bacterium belonging to genus Acidipila is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).
[13] The method according to [11] or [12], wherein the filamentous fungus is selected from the group consisting of fungi of genus Aspergillus, fungi of genus Mucor, and fungi of genus Trichophyton.
[14] The method according to [11] or [12], wherein the yeast is selected from the group consisting of fungi of genus Saccharomyces, fungi of genus Candida, fungi of genus Malassezia, and fungi of genus Rhodotorula.
[15] The method according to any one of [11] to [14], wherein the antifungal substance derived from a bacterium belonging to genus Acidipila is a culture or a culture supernatant of the bacterium, or a concentrate or an extract of the culture or culture supernatant.
[16] A use of a culture or a culture supernatant of a bacterium belonging to genus Acidipila, or a concentrate or an extract of the culture or culture supernatant, as an antifungal agent targeting a yeast or a filamentous fungus.
[17] The use according to [16], wherein the bacterium belonging to genus Acidipila is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).
[18] The use according to [16] or [17], wherein the filamentous fungus is selected from the group consisting of fungi of genus Aspergillus, fungi of genus Mucor, and fungi of genus Trichophyton.
[19] The use according to [16] or [17], wherein the yeast is selected from the group consisting of fungi of genus Saccharomyces, fungi of genus Candida, fungi of genus Malassezia, and fungi of genus Rhodotorula.

### EFFECTS OF THE INVENTION

The present invention provides a novel antifungal substance derived from a bacterium belonging to the genus Acidipila. The antifungal substance is useful as an effective ingredient of, for example, pharmaceuticals for treating diseases caused by fungi, cosmetics, detergents, food additives (preservatives etc.), disinfectants, and the like.

### BRIEF DESCRIPTION OF THE DRAWINGS

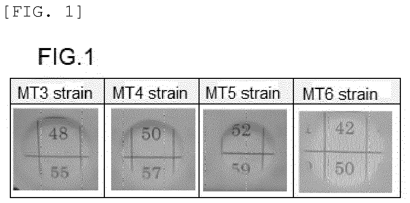

FIG. 1 shows results of antifungal activity tests using the antifungal substances of the present disclosure.

### MODE FOR CARRYING OUT THE INVENTION

The antifungal substance of the present disclosure is a substance that a bacterium belonging to the genus Acidipila produces as a secondary metabolite. The antifungal substance means a substance having antifungal activity. As used herein, the "antifungal activity" refers to the ability to suppress or inhibit the growth of fungi. The antifungal activity can be determined by a known method in the art. Examples of the known method include a spot-on-lawn method, a direct method, a paper disc method, and a minimum inhibitory concentration (MIC) method.

Fungi targeted by the antifungal substance of the present disclosure are not particularly limited. The antifungal substance of the present disclosure has antifungal activity against a variety of fungi including various yeast and filamentous fungi. The antifungal substance of the present disclosure preferably has antifungal activity against yeast and filamentous fungi. Examples of yeast include, but not limited to, microorganisms belonging to genus Saccharomyces, genus Zygosaccharomyces, genus Candida, genus Cryptococcus, genus Malassezia, genus Rhodotorula, and genus Pneumocyctis. Preferable examples thereof include microorganisms of the genus Saccharomyces, the genus Candida, the genus Malassezia, and the genus Rhodotorula. Specific examples of yeast include, but not limited to, *Saccharomyces cerevisiae, Zygosaccharomyces rouxii, Candida albicans, Candida glabrata, Candida tropicalis, Candida auris, Cryptococcus neoformans, Malassezia furfur, Rhodotorula glutinis,* and *Pneumocystis jirovecii,* and preferably *Saccharomyces cerevisiae, Candida albicans, Candida glabrata, Candida tropicalis, Malassezia furfur,* and *Rhodotorula glutinis.* Examples of filamentous fungi include, but not limited to, microorganisms belonging to genus Trichophyton, genus Aspergillus, genus Mucor, genus Penicillium, genus Chaetomium, genus Cladosporium, genus Aureobasidium, genus Gliocladium, and genus Paecilomyces. Preferable examples thereof include microorganisms of the genus Trichophyton, the genus Mucor, and the genus Aspergillus. Specific examples of filamentous fungi include, but not limited to, *Trichophyton rubrum, Trichophyton mentagrophytes, Aspergillus niger, Aspergillus flavus, Aspergillus fumigatus, Aspergillus terreus, Mucor mucedo, Penicillium funiculosum, Chaetomium globosum, Cladosporium cladosporioides, Aureobasidium pullulans, Gliocladium virens,* and *Paecilomyces variotii,* and preferably *Trichophyton rubrum, Trichophyton mentagrophytes, Aspergillus niger, Aspergillus flavus, Aspergillus fumigatus, Aspergillus terreus,* and *Mucor mucedo.*

In the present disclosure, the bacterium belonging to the genus Acidipila may be any bacterial species or strain belonging to the genus Acidipila. For example, known bacterial species including, but not limited to, *Acidipila dinghuensis* and *Acidipila rosea* may be used. A newly isolated bacterium belonging to the genus Acidipila may be also used. The bacterium belonging to the genus Acidipila can be isolated, for example, from soil. Isolation and screening of the bacterium may be a known method, which can be appropriately selected by a person skilled in the art. For example, the bacterium may be cultured in a known solid medium, such as Medium 1426 ("SSE/HD 1:10" medium) published by DSMZ (German Collection of Microorganisms and Cell Cultures) GmbH, for example at 25°C to 35°C for 24 hours to 30 days, preferably at 28°C to 30°C for 7 to 14 days. The culture conditions may be appropriately determined by a person skilled in the art depending on the emergence and growth of bacterial cells. In the present disclosure, it was found for the first time that a bacterium belonging to the genus Acidipila produces an antifungal substance. Therefore, the bacterium belonging to the genus Acidipila used in the present disclosure has the ability to produce an antifungal substance.

Further in the present disclosure, four new bacterial strains of the genus Acidipila were isolated, and then identified as new species. These strains were named Acidipila sp. MT3 strain, MT4 strain, MT5 strain, and MT6 strain, respectively, and deposited at the National Institute of Technology and Evaluation Patent Microorganisms Depositary (2-5-8 Kazusakamatari, Kisarazu-shi, Chiba, Japan) on March 1, 2022 (Accession No. NITE BP-03614, Accession No. NITE BP-03615, Accession No. NITE BP-03616, and Accession No. NITE BP-03617, respectively). Therefore, in one embodiment of the present disclosure, Acidipila sp. strains MT3, MT4, MT5, or MT6 are used.

The antifungal substance of the present disclosure is a secondary metabolite produced by culturing a bacterium belonging to the genus Acidipila. A method for culturing a bacterium belonging to the genus Acidipila is not particularly limited. The bacterium may be subjected to liquid culture or solid culture, and liquid culture is preferred. An medium used for culture may be any medium generally used for bacterial culture. Examples of the medium include, but not limited to, Medium 1426 ("SSE/HD 1:10" medium) published by DSMZ (German Collection of Microorganisms and Cell Cultures) GmbH. Culture temperature and time may be appropriately determined by a person skilled in the art. For example, the bacterium may be cultured at 25°C to 35°C for 24 hours to 30 days, preferably at 28°C to 30°C for 7 to 14 days.

The antifungal substance of the present disclosure can be obtained from a culture or culture supernatant of a bacterium belonging to the genus Acidipila. The antifungal substance of the present disclosure may be a culture or culture supernatant itself of a bacterium belonging to the genus Acidipila, or a concentrate or extract of the culture or culture supernatant. The culture supernatant can be obtained by a conventional method. For example, the culture supernatant may be obtained by removing bacterial cells from the cell culture by centrifugation or the like. The culture supernatant may be further subjected to concentration, extraction, and/or purification. The concentration, extraction and purification can be performed by conventional methods. For the concentration and extraction, for example, vacuum concentration, various filtration including filtration via a filter, ultrafiltration etc., extraction with an organic solvent such as methanol etc., various chromatography methods including column chromatography, high-performance liquid chromatography etc., liquid-liquid extraction using chloroform or ethyl acetate, etc. may be used. For the purification, for example, distillation, back extraction, column chromatography, recrystallization, etc. may be used.

The present disclosure further provides an antifungal agent comprising the antifungal substance of the present disclosure as an effective ingredient. The antifungal agent is in the form of a composition. Examples of the composition include a pharmaceutical composition, a cosmetic composition (for example, shampoo, conditioner, bath salt, etc.), a detergent composition (for example, detergent for house, laundry detergent, soap, etc.), a food additive composition, and a disinfectant composition (which is applied, for example, to food products, cooking utensils, walls and floors of buildings including hospitals, and furniture including desks). The antifungal agent may contain an additional component, such as a pharmaceutically or sitologically acceptable carrier, excipient, solvent, stabilizer, solubilizer etc., in addition to the antifungal substance of the present disclosure. The antifungal agent may further contain another antifungal substance in addition to the antifungal substance of the present disclosure. The antifungal agent may be in any dosage form, which may be appropriately selected depending on the purpose of use. Examples of the dosage form include, but not limited to, a lotion, cream, a poultice, an ointment, a tablet, powder, a capsule, granulated powder, syrup, an infusion, an injection, a granule, powder, a solution, a suspension, paste, a spray, and a block. When the antifungal agent is a pharmaceutical composition, the pharmaceutical composition can be used as a therapeutic or preventive agent for mycoses. Examples of mycoses include cutaneous mycoses and deep mycoses. Specific examples of mycoses include, but not limited to, ringworm, Malassezia dermatitis, candidiasis, cryptococcosis, aspergillosis, zygomycosis, and pneumocystis, and preferably ringworm, Malassezia dermatitis, candidiasis, aspergillosis, and zygomycosis. An administration route of the pharmaceutical composition may be appropriately selected according to the therapeutic purpose. Examples of the administration route include, but not limited to, oral administration, transdermal administration, nasal administration, and administration by injection. A dose of the pharmaceutical composition is appropriately determined by a person skilled in the art. When the antifungal agent of the present disclosure is a cosmetic composition or a food additive composition, for example, the composition can be used as a preservative. When the antifungal agent of the present disclosure is a detergent composition or a disinfectant composition, for example, the composition can be used for removing fungi from or preventing fungi from tainting facilities of buildings and houses (e.g., walls, floors, wet areas such as bathrooms, etc.), cooking utensils, laundry, etc. An amount of the antifungal agent to be used can be appropriately determined according to the purpose of use, usage conditions, and the like.

The antifungal substance of the present disclosure may be used anywhere inhibition of fungal growth is desired. The growth of a fungus is inhibited by bringing the antifungal agent into contact with the fungus. Therefore, the present disclosure provides a method for inhibiting the growth of a fungus, which comprises bringing an antifungal substance derived from a bacterium belonging to the genus Acidipila into contact with the fungus. As used herein, "inhibition" includes suppression and inhibition of fungal growth, and killing of fungi. Examples of a situation where inhibition of fungal growth is desired include, but not limited to, a situation where the treatment or prevention of diseases caused by fungi such as mycoses is needed, a situation where the prevention of fungal contamination, deterioration or spoilage of foods and cosmetics is needed, and a situation where the decontamination or prevention of fungal contamination in facilities of houses and buildings, cooking utensils, laundry, etc. is needed.

A method of bringing the antifungal substance into contact with a fungus may be appropriately selected depending on the purpose of use of the antifungal substance (that is, depending on the situation where inhibition of the growth of the fungus is desired). For example, the antifungal substance may be administered, added, applied, or sprayed to a target subject, or the target subject may be immersed in the antifungal substance. The target subject is appropriately selected depending on the intended use of the antifungal substance. For example, for the purpose of treating or preventing diseases such as mycoses, the antifungal substance may be administered to animals such as human. For example, for the purpose of preventing fungal contamination, deterioration or spoilage of foods, cosmetics, etc., the antifungal substance may be added to foods, cosmetics, etc. For example, for the purpose of decontaminating or preventing fungal contamination in facilities of houses or buildings, etc., the antifungal substance may be added, applied, or sprayed to facilities of houses or buildings, etc., or the facilities, etc. may be immersed in the antifungal substance. In the method for inhibiting the growth of a fungus, the antifungal substance may also be the antifungal agent, and the antifungal agent may be in the form of the composition as described above. The method for inhibiting the growth of a fungus comprises administering, adding, applying or spraying an effective amount of the antifungal substance or the antifungal agent to the subject or immersing the subject in an effective amount of the antifungal substance or the antifungal agent. Therefore, another aspect of the present disclosure provides a method for treating or preventing a mycosis which comprises administering to a patient an effective amount of an antifungal substance derived from a bacterium belonging to the genus Acidipila or an antifungal agent comprising the antifungal substance. As still another aspect of the present disclosure, provided is a method for preventing fungal contamination, deterioration or spoilage of foods or cosmetics which comprises adding to the foods or cosmetics an effective amount of an antifungal substance derived from a bacterium belonging to the genus Acidipila or an antifungal agent comprising the antifungal substance. As still another aspect of the present disclosure, provided is a method for decontaminating or preventing fungal contamination in facilities of houses or buildings, cooking utensils, laundry, etc. which comprises adding, applying, or spraying an effective amount of an antifungal substance derived from a bacterium belonging to the genus Acidipila or an antifungal agent comprising the antifungal substance to the facilities of houses or buildings, cooking utensils, laundry, etc., or immersing the facilities of houses or buildings, cooking utensils, laundry, etc. in an effective amount of an antifungal substance derived from a bacterium belonging to the genus Acidipila or an antifungal agent comprising the antifungal substance.

Hereinafter, the present invention is explained in more detail by way of Examples which the present invention is not limited to.

### EXAMPLE 1

### Example 1: Identification of novel Acidipila bacteria

Genomic sequences of four bacterial strains belonging to the genus Acidipila that were isolated from soil were determined by a conventional method, and subjected to 16S rDNA analysis, whole genomic analysis, and average nucleotide identity (ANI) analysis. In the 16S rDNA analysis, when a test bacterium has a homology of 98.7% or less with a standard strain, the bacterium is regarded as a new species. In the ANI analysis, when a test bacterium has an ANI value of 95% or less, the bacterium is regarded as a new species. Isolation of the bacterium from soil was carried out by culturing on 1.5wt% agar medium of Medium 1426 "SSE/HD 1:10" medium published by DSMZ (German Collection of Microorganisms and Cell Cultures) GmbH at 30°C for 2 weeks.

### <16S rDNA analysis>

The bacterial cells were lysed with achromopeptidase (manufactured by Fujifilm Wako Pure Chemical Industries, Ltd.). A DNA was extracted from the lysed bacterial cell by a conventional method, amplified by PCR using PrimeSTAR HS DNA Polymerase (manufactured by Takara Bio Inc.), subjected to cycle sequencing using BigDye Terminator v3.1 Cycle Sequencing Kit (manufactured by Applied Biosystems), and then sequenced using ChromasPro1.7 (manufactured by Technelysium). Homology analysis regarding about 600 bases of the V1 to V4 regions of 16S rDNA was performed by comparison with the NCBI international nucleotide sequence database. Then, a simple molecular phylogenetic tree was made, and species was identified. As a result, all the four isolated strains had the highest sequence homology with *Acidipila dinghuensis* among known species of the genus Acidipila, and their 16S rDNA homology showed that these four strains were new species of the genus Acidipila (Table 1). These four new strains were named MT3, MT4, MT5 and MT6, respectively.

### <Whole genomic analysis>

The MT3, MT4, and MT5 strains were subjected to whole genomic analysis, and their nucleotide sequences were used to perform ANI analysis. A DNA was extracted from each strain using DNeasy Blood & Tissue Kit (manufactured by Qiagen), and a library was prepared using Illumina DNA Prep (M) Tagmentation kit. Nextera DNA CD Indexes kit was used as an index kit. Sequencing (301 bp paired-end analysis) was performed using a next-generation sequencer (MiSeq, Illumina). Genome assembly was performed using Platanus B to determine a nucleotide sequence. The resulting assemblies were evaluated using QUAST and checkM.

### <ANI analysis>

Next, ANI values were calculated using EZbiocloud (https://www.ezbiocloud.net/tools/ani), and homology between strains was analyzed. In the ANI analysis, the genomic sequence of a target strain is fragmented into 1,020-bp fragments on computer, homology searches of each fragment against the genomic sequence of a comparative strain are performed to obtain homology values, and an ANI value between the genomic sequences is calculated from the average homology value. *Acidipila dinghuensis* DHOF10 and *Acidipila dinghuensis* CGMCC 1.13007 registered in the NCBI International Nucleotide Sequence Database were used as comparative strains to perform ANI analysis. As a result, all the target strains tested had an ANI value less than 95% against the comparative strains, and thus it was found that the target strains were new species (Table 1). Since the MT3, MT4 and MT5 strains differed in homology to the comparative strains from one another, it was found that the MT3, MT4 and MT5 strains probably had different genetic information from one another. In addition, *Acidipila rosea* (DSM103428) was used as a comparison strain to perform ANI analysis. As a result, all the target strains tested had an ANI value less than 95% against the comparative strain (Table 1).

**[Table 1]**

| | 16S rDNA anal ysis | | Whole genomic analysis | |
|---|---|---|---|---|
| Strain | Spec hav hom cies ing high ology | Homolo gy | Comparative strain | ANI value |
| MT3 | *Acidipila dinghuensis* | 96.5% | *Acidipila dinghuensis* | 71.5% |
| | | | DHOF10 | |
| | | | *Acidipila dinghuensis* | 71.3% |
| | | | CGMCC 1.13007 | |
| | | | *Acidipila rosea* | 71.6% |
| | | | DSM 103428 | |
| MT4 | *Acidipila dinghuensis* | 97.2% | *Acidipila dinghuensis* | 71.4% |
| | | | DHOF10 | |
| | | | *Acidipila dinghuensis* | 71.5% |
| | | | CGMCC 1.13007 | |
| | | | *Acidipila rosea* | 71.6% |
| | | | DSM 103428 | |
| MT5 | *Acidipila dinghuensis* | 97.2% | *Acidipila dinghuensis* | 71.2% |
| | | | DHOF10 | |
| | | | *Acidipila dinghuensis* | 71.1% |
| | | | CGMCC 1.13007 | |
| | | | *Acidipila rosea* | 71.5% |
| | | | DSM 103428 | |
| MT6 | *Acidipila dinghuensis* | 97.2% | | |

### EXAMPLE 2

### Example 2: Culture of Acidipila bacteria in production medium and preparation of culture concentrate

A medium for antifungal substance production was prepared, and 100 mL of the medium was put into a 500 mL Erlenmeyer flask and autoclaved at 121°C for 20 min. After the autoclave sterilization, 1 mL of a bacterial strain that had been liquid-cultured in advance was added into the flask. This work was performed under an aseptic condition in a safety cabinet. The flask was subjected to stirring culture on a shaker at 27°C and 210 rpm for 6 days to allow the bacterium to produce a secondary metabolite. Then, an equal amount (100 mL) of methanol was added to a culture solution, followed by stirring at 210 rpm for 30 minutes. After the stirring, the culture solution was centrifuged at 3000 rpm for 10 minutes to remove bacterial cells. A supernatant was transferred into a 1 L eggplant flask, and concentrated using an evaporator in a hot water bath of 40°C. A vacuum was gradually drawn up to 80 hPa to remove methanol and obtain a culture concentrate sample.

Acidipila sp. MT3, MT4, MT5, and MT6 strains were used as the bacterial strain. For liquid culture (preculture), the bacterial strains were grown in a YD medium (1% glucose, 1% yeast extract) at 30°C for 6 days to an OD600 of 1 to 1.5. For antifungal substance production, the bacterial strains were cultured in the Medium 1426 at 28°C for 14 days.

### EXAMPLE 3

### Example 3: Antifungal activity test (Spot-on-lawn assay)

*Saccharomyces cerevisiae* (NBRC10505) was used as a test fungus. The fungus was inoculated into an MRS (manufactured by Becton, Dickinson and Company) liquid medium and cultured with stirring at 30°C and 150 rpm for 1 day to prepare a test fungal solution. A test tube containing 7 mL of an MRS soft agar medium (agar 0.5%) was sterilized by an autoclave (105°C/10 minutes), and then cooled to 55 to 60°C. To the soft agar medium, 70 µL of the test fungal solution prepared by culturing the test fungus in the liquid medium as described above was added, and then stirred using a stirrer. The soft agar medium was poured onto an MRS agar medium plate (1.5% agar) that was prepared in advance to prepare a medium for antifungal activity test. After addresses on the plate were determined, 10 µL of the culture concentrate sample obtained in Example 2 was spotted. The plate was cultured at 30°C for 24 hours and visually observed. In this experiment, an inhibition circle can be seen in the presence of the antifungal activity.

As a result, when any of the culture concentrate samples of the four bacterial strains was spotted, an inhibition circle was observed (FIG. 1). Therefore, it was found that the culture concentrates of Acidipila bacteria have antifungal activity.

### EXAMPLE 4

### Example 4: Antifungal activity test against various fungi

As test yeast, *Candida albicans, Malassezia furfur,* and *Rhodotorula glutinis* were used. As test filamentous fungi, *Trichophyton rubrum, Aspergillus niger,* and *Mucor mucedo* were used. *Candida albicans, Aspergillus niger,* and *Mucor mucedo* are known as pathogens of deep mycoses Candidiasis, Aspergillosis, and Zygomycosis, respectively. *Malassezia furfur* and *Trichophyton rubrum* are known as pathogens of cutaneous mycoses Malassezia dermatitis and ringworm, respectively. *Rhodotorula glutinis* is not pathogenic, and is known as a red mold that is found in water areas such as a bathroom, etc.

Each test fungus was inoculated into a liquid medium and cultured with stirring at 30°C and 150 rpm for 1 day to prepare each test fungal solution. A test tube containing 7 mL of a soft agar medium (agar 0.5%) suited to each test fungus was sterilized by an autoclave (105°C/10 minutes), and then cooled to 55 to 60°C. To the soft agar medium, 70 µL of the test fungal solution prepared by culturing the test fungus in the liquid medium as described above was added, and then stirred using a stirrer. The soft agar medium was poured onto an agar medium plate (1.5% agar) that was prepared in advance to prepare a medium for antifungal activity test. Table 2 shows the composition of the liquid medium used for each test fungus.

**[Table 2]**

| *Trichophyton rubrum* | | | | *Aspergillus niger*/*Mucor mucedo* | | |
|---|---|---|---|---|---|---|
| Peptone | 10 | g | | Potato | 200 | 9 |
| Glucose | 40 | g | | Sucrose | 20 | 9 |
| Distilled water | 1 | L | | Distilled water | 1 | L |

| *Candida albicans*/*Rhodotorula glutinis* | | | | *Malassezia furfur* | | |
|---|---|---|---|---|---|---|
| Glucose | 10 | g | | Glucose | 10 | 9 |
| Peptone | 5 | g | | Peptone | 5 | 9 |
| Yeast extract | 3 | g | | Yeast extract | 3 | 9 |
| Malt extract | 3 | g | | Malt extract | 3 | 9 |
| Distilled water | 1 | L | | Olive oil | 10 | 9 |
| | | | | Distilled water | 1 | L |

A ϕ5 mm paper disc was placed on the medium for antifungal activity test, and 50 µL of the culture concentrate sample obtained in Example 2 was spotted (paper disc method) . The plate was cultured at 30°C for 24 hours and visually observed. A hole of ϕ5 mm was punched out of the medium for antifungal activity test, and 250 µL of the culture concentrate sample obtained in Example 2 was spotted (agar well diffusion method). The plate was cultured at 30°C for 24 hours and visually observed. In both experiments, an inhibition circle can be seen in the presence of the antifungal activity. When an inhibition circle was visually observed, the size of the inhibition circle was measured. The size of the inhibition circle was determined by measuring with a scale the distance from the circumference of the paper disc (in the case of the paper disc method) or hole (in the case of the agar well diffusion method) lying in the center of the inhibition circle to the circumference of the inhibition circle, in terms of the longest radius of the inhibition circle observed. When a double zone of inhibition was observed, the size of an inner inhibition circle was measured. Results are shown in Table 3. In Table 3, "no activity" means that no inhibition circle was visually observed.

**[Table 3]**

| Test fungi | Size of inhibition circle | | | | | | | |
|---|---|---|---|---|---|---|---|---|
| | MT3 strain | | MT4 strain | | MT5 strain | | MT6 strain | |
| | 50 µL | 250 µL | 50 µL | 250 µL | 50 µL | 250 µL | 50 µL | 250 µL |
| *Trichophyton rubrum* | 6.5 mm | Not tested | 6.0 mm | Not tested | 6.0 mm | Not tested | 6.5 mm | Not tested |
| NBRC 5467 | | | | | | | | |
| *Aspergillus niger* | No activity | 5 mm | No activity | 4.5 mm | No activity | 4.5 mm | No activity | 5 mm |
| NBRC 33023 | | | | | | | | |
| Mucor *mucedo* | No activity | 1.5 mm | No activity | 1.5 mm | No activity | 1.5 mm | No activity | 1.5 mm |
| NHRC 5776 | | | | | | | | |
| *Candida albicans* | No activity | 0.5 mm | No activity | 0.5 mm | No activity | 0.5 mm | No activity | 0.5 mm |
| NBRC 1594 | | | | | | | | |
| *Rhodotorula glutinis* | No activity | 5 mm | No activity | 5 mm | No activity | 5 mm | No activity | 5 mm |
| NBRC 1241 | | | | | | | | |
| Malassezia *furfur* | No activity | 2 mm | No activity | 1.8 mm | No activity | 1.8 mm | No activity | 2 mm |
| NaRC 10988 | | | | | | | | |

As a result, inhibition circles were observed against all of the test fungi. Particularly large inhibition circles were observed against *Trichophyton rubrum,* indicating strong antifungal activity against Trichophyton fungi.

## Claims

1. An antifungal agent, comprising an antifungal substance derived from a bacterium belonging to genus Acidipila and targeting a yeast or a filamentous fungus.

2. The antifungal agent according to claim 1, wherein the bacterium belonging to genus Acidipila is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).

3. The antifungal agent according to claim 1 or 2, wherein the filamentous fungus is selected from the group consisting of fungi of genus Aspergillus, fungi of genus Mucor, and fungi of genus Trichophyton.

4. A method for producing an antifungal agent targeting a yeast or a filamentous fungus, the method comprising culturing a bacterium belonging to genus Acidipila.

5. The method according to claim 4, wherein the bacterium belonging to genus Acidipila is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).

6. A bacterium which is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).

7. A method for inhibiting the growth of a fungus which is a yeast or a filamentous fungus, the method comprising bringing an antifungal substance derived from a bacterium belonging to genus Acidipila into contact with the yeast or filamentous fungus.

8. The method according to claim 7, wherein the bacterium belonging to genus Acidipila is Acidipila sp. MT3 strain (Accession number NITE BP-03614), MT4 strain (Accession number NITE BP-03615), MT5 strain (Accession number NITE BP-03616), or MT6 strain (Accession number NITE BP-03617).

9. The method according to claim 7 or 8, wherein the filamentous fungus is selected from the group consisting of fungi of genus Aspergillus, fungi of genus Mucor, and fungi of genus Trichophyton.
